Europäisches Patentamt

European Patent Office

Office européen des brevets

(11) Publication number: **0 079 717**
**B1**

## EUROPEAN PATENT SPECIFICATION

(45) Date of publication of patent specification: **25.09.85**

(21) Application number: **82305832.6**

(22) Date of filing: **02.11.82**

(51) Int. Cl.⁴: **G 01 N 21/82,** G 01 N 33/543

(54) **A method of judging a particle agglutination reaction and a reaction vessel for use in the method.**

(30) Priority: **02.11.81 JP 176250/81**

(43) Date of publication of application:
**25.05.83 Bulletin 83/21**

(45) Publication of the grant of the patent:
**25.09.85 Bulletin 85/39**

(84) Designated Contracting States:
**DE FR GB**

(56) References cited:
**DE-A-2 905 558**
**DE-A-3 033 870**
**JP-A-56 002 559**
**JP-A-56 002 563**
**JP-A-56 002 564**
**US-A-4 240 751**
**US-A-4 262 196**

**Patent Abstracts of Japan, vol. 5, no. 48, 7 April
1981**
**Patent Abstracts of Japan Vol. 5, no. 48, 7 April
1981**
**Patent Abstracts of Japan Vol. 5, no. 48, 7 April
1981**

(73) Proprietor: **OLYMPUS OPTICAL CO., LTD.**
**43-2, 2-chome, Hatagaya Shibuya-ku**
**Tokyo 151 (JP)**

(72) Inventor: **Kano, Tokio**
**No. 42-26, 2-Chome, Fujimidai**
**Kunitachi City Tokyo (JP)**
Inventor: **Tamagawa, Akira**
**No. 224, Hirayama-Jutaku No. 7-7, 1-Chome**
**Higashihirayama Hino City Tokyo (JP)**

(74) Representative: **Fane, Christopher Robin King
et al**
**HASELTINE LAKE & CO. Hazlitt House 28
Southampton Buildings Chancery Lane
London, WC2A 1AT (GB)**

Courier Press, Leamington Spa, England.

## Description

The present invention generally relates to an immunological agglutination analysis and more particularly to a technique for testing or judging the existence of particular antigens or antibodies due to a particle agglutination reaction.

Heretofore, there have been proposed various types of such immunological agglutination judging methods for determining blood types and various kinds of antigens and antibodies by detecting photoelectrically an agglutinated particle pattern or a non-agglutinated particle pattern formed by descending particles on a conically inclined interior bottom surface of a reaction vessel, while the reaction vessel is maintained substantially stationary. It has been further proposed to provide an array of reaction vessels, each having a conical interior bottom surface, by forming a base plate with a matrix of depressions so as to constitute a microplate. In such an immunological agglutination testing method using a reaction vessel having a conical bottom surface, when there is an agglutination reaction the agglutinated particles are deposited on the inclined surface just like snow to form a uniformly deposited pattern, while in the case of a non-agglutination reaction the particles roll down along the inclined surface and are collected at the lowermost portion, i.e. at the apex of the cone, to form a collected pattern.

In a known method for monitoring photoelectrically the uniformly deposited pattern and the collected pattern and distinguishing therebetween, an image of the particle pattern on the conical bottom surface is formed on an image plane on which two light receiving elements are arranged in such a manner that the first element receives an image of the lowermost portion of the conical bottom surface and the second element receives an image of a higher portion of the inclined bottom surface, surrounding said lowermost portion, and a difference between output signals of these elements is compared with a predetermined reference level to detect the existence of the particle agglutination reaction. An example of such a method is disclosed in JP—A—56-2559.

In the known method, when the concentration of a particle suspension and an amount of the particle suspension delivered into the reaction vessel are varied, the outputs of the light detectors also vary, so that accurate detection can no more be effected. For instance, in the case of determining blood types, a sample of whole blood is first centrifuged to form a blood cell sediment and a given amount of the sedimented blood cells is delivered into the reaction vessel. In this case, the degree of sedimentation of blood cells during the centrifuging might be affected by variation of various conditions of centrifuging, and of mechanical shock and vibration to which the sedimented blood cells are subjected after centrifuging, and differences in viscosity and specific gravity of blood cells and blood plasma.

Therefore, even if a predetermined amount of blood cell sediment is delivered, variations may occur in the amount and/or concentration of delivered blood cells. Further, such a variation might be produced owing to a fluctuation of the delivery mechanism.

In the above-mentioned known method, the uniformly deposited pattern might be erroneously determined as a partly deposited pattern, produced by particles having a weak agglutination reaction, when the particle concentration is too high, because particles which could not be fully agglutinated fall down into the lowermost portion. Contrary to this, when the particle concentration is too low, the partly deposited pattern might be misjudged as the uniformly deposited pattern, and the collected pattern might be erroneously judged as the uniformly deposited pattern, because the amount of particles deposited at the lowermost portion is too small. Moreover, when the particle concentration is extremely small, even in the case of the non-agglutination reaction, since the amount of particles collected in the lowermost portion is very small, the collected pattern can hardly be distinguished from the uniformly deposited pattern.

It is desirable to reduce the likelihood of occurrence of such erroneous judgements.

In accordance with one aspect of the present invention there is provided a method of judging the occurrence of a particle agglutination reaction in a test liquid sample, wherein the sample is placed in a reaction vessel having a main interior bottom surface portion that is inclined to the horizontal, and photoelectric means are used to produce a detection signal which is dependent upon the difference between deposited particle concentration at a lowermost part of the said bottom surface portion and deposited particle concentration at a higher part of that surface portion and which is used as a basis for the said judgement, characterised in that the reaction vessel has a further interior bottom surface portion, which extends in a horizontal plane, photoelectric means are used to produce an auxiliary signal which is dependent upon deposited particle concentration at the said further bottom surface portion, and the said auxiliary signal is used to control the making of the said judgement.

In accordance with another aspect of the present invention there is provided a reaction vessel, for use in judging the occurrence of a particle agglutination reaction in a test liquid sample contained in the vessel, having a main interior bottom surface portion that is inclined to the horizontal for receiving a pattern of deposited particles which is indicative of the degree of agglutination; characterised in that the vessel has a further interior bottom surface portion, which extends in a horizontal plane, for receiving deposited particles to afford an indication of particle concentration and/or the total quantity of particles in the test liquid sample.

Reference will now be made, by way of example, to the accompanying drawings, wherein:

Fig. 1 shows an axial sectional view of a reaction vessel for use in accordance with an embodiment of the present invention;

Fig. 2 shows a perspective view of a reaction vessel plate for use in accordance with an embodiment of the present invention; and

Fig. 3 shows a schematic view illustrating an apparatus for making use of the reaction vessel of Fig. 1.

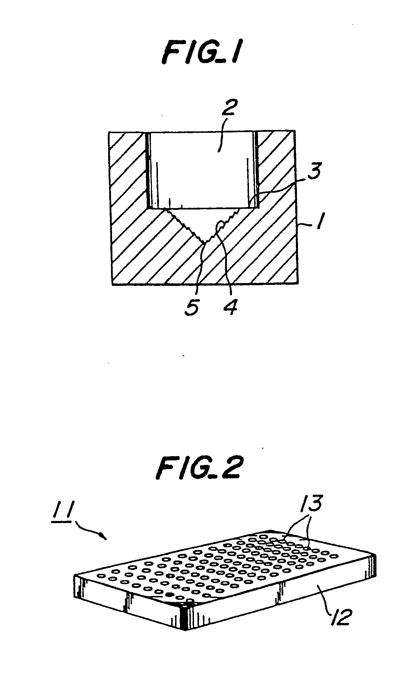

Fig. 1 shows a reaction vessel which comprises a vessel body 1 made of transparent plastics having an opening 2 through which sample and reagent are delivered into the vessel. The interior bottom surface of the vessel comprises a main surface portion 4 that is inclined to the horizontal, being conical in form, and an adjoining further portion 3 that is annular and extends in a horizontal plane. The further bottom surface portion 3 adjoins an upright inner wall surface bounding the opening 2, and the main bottom surface portion 4 is formed with a number of regularly spaced steps from its uppermost edge to its central lowermost portion (apex) 5. The steps may be replaced by grooves, projections or recesses.

Fig. 2 shows a reaction vessel plate 11 which comprises a base plate 12, made of transparent plastics, formed with a number of recesses 13, constituting reaction chambers, arrayed in a matrix form. Each recess 13 has an interior bottom surface consisting of a ring-shaped flat portion and a conically inclined portion having a number of regular steps just as in the vessel of Fig. 1, so that the plate 11 provides a matrix of such vessels constructed integrally with one another.

In the reaction vessel of Fig. 1 descending particles are deposited on the flat surface portion 3 to an extent which depends upon the total quantity of particles, and/or the concentration of the test liquid, delivered into the reaction vessel. On the inclined surface portion 4, at first a stable base layer of particles is formed owing to the steps formed in that surface portion and then, when the antigen-antibody reaction occurs, the agglutinated particles are uniformly deposited on that stable base layer to form the uniformly deposited particle pattern. In contrast, when the antibody-antigen reaction does not occur, the descending particles roll down along the base layer and are collected at the central lowermost portion 5 to form the collected particle pattern.

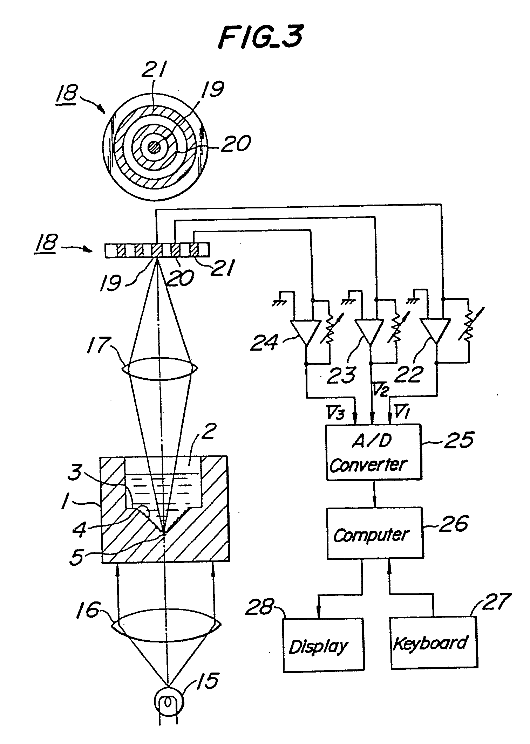

Fig. 3 shows an apparatus for making use of the reaction vessel 1 shown in Fig. 1. A light flux emitted from a light source 15 is collimated by a collimator lens 16 and directed through the bottom of the reaction vessel 1. An image of a particle pattern formed on the interior bottom surface 3, 4 and 5 of the reaction vessel is projected by an imaging lens 17 onto a light detector 18. The light detector 18 comprises a light receiving element 19 for receiving the image of the central lowermost portion 5, a second light receiving element 20 for receiving an image from a higher (middle) part of the main bottom surface portion 4, and a third light receiving element 21 for receiving the image of the flat bottom surface portion 3. Accordingly, these light receiving elements 19, 20 and 21 are arranged concentrically with respect to an optical axis passing through the central portion 5 of the reaction vessel. Photoelectrically converted output signals from these light receiving elements 19, 20 and 21 are supplied, via pre-amplifiers 22, 23 and 24 respectively, to an A—D converter 25 including a multiplexer.

The analog output signals are successively converted into digital signals which are then supplied to a computer 26 to which are also supplied, from a keyboard 27, standard values as will be explained below. The computer 26 carries out a given calculation with the aid of the digital signals supplied from the A/D converter 25 and the standard values supplied from the keyboard 27 to judge the particle pattern, and its result is displayed on a display device 28.

Examples of processes for judging the particle pattern will now be explained.

Example 1

In this example, upper and lower reference values $DR_1$ and $DR_2$, to be compared with the auxiliary signal $V_3$ supplied from the light receiving element 21, have been previously stored in a memory of the computer 26 by means of the keyboard 27. These upper and lower reference values $DR_1$ and $DR_2$ correspond respectively to upper and lower limit values of the auxiliary signal $V_3$ which will be obtained when the total quantity of particles, and/or particle concentration of the test liquid, is within a normal range. These reference values $DR_1$ and $DR_2$ can be determined experimentally.

When $V_3 > DR_1$ or $V_3 < DR_2$, it can be judged that the particle concentration, and/or total quantity of particles, is out of the normal range. In such a case, no determination of the particle pattern is made, and a suitable mark such as "ABNORMAL" is displayed on the display device 28.

When $DR_1 \leqq V_3 \leqq DR_2$, the particle concentration, and/or total quantity of particles, can be recognized to be within the normal range. The particle pattern can then be judged with the aid of the output signals $V_1$ and $V_2$ supplied from the two light receiving elements 19 and 20 respectively, in a manner similar to that of the known method. That is to say, firstly a difference $\Delta V = V_2 - V_1$ is derived to serve as a detection signal, and this detection signal is then compared with judgement standard upper and lower limits U.L. and L.L. (U.L. > 1L.L.) which have been previously stored in the computer 26 via the keyboard 27. When the particle agglutination reaction occurs, a difference in an optical density between the main bottom surface portion 4 and the lowermost central portion 5 becomes very small and $\Delta V$ becomes smaller than the lower limit value L.L.

Therefore, when $\Delta V$<L.L., it is recognized that the agglutination reaction has occurred and a mark such as "+" is displayed on the display device 28. In contrast, when non-agglutination has occurred, the particles descending on the main bottom surface portion 4 fall down into the lowermost central portion 5 to form the collected pattern. In this case, $\Delta V$ becomes larger than the upper limit value U.L. Therefore, when $\Delta V$>U.L., a mark "−" is displayed on the display device 28.

When the agglutination force is weak and a partly deposited particle pattern is formed, U.L.$\geqq\Delta V\geqq$L.L. is obtained. Then a "?" mark is displayed, because in such a case it is difficult to effect a reliable judgement.

In the present example, the possibilities of making erroneous judgements is reduced, as compared with the prior art, because when the particle concentration and/or total quantity of particles are too large or too small, the judgement is not effected.

Example 2

In this example, in the computer 26 there has been previously stored a reference value $V_{30}$ by means of the keyboard 27. This reference value $V_{30}$ corresponds to a value of the signal $V_3$ which is obtained when the particle concentration and/or total quantity of particles is within a predetermined standard range and can be determined experimentally. Firstly, a correction coefficient $\gamma=V_3/V_{30}$ is calculated in the computer 26, and then a product of the coefficient $\gamma$ and the difference $\Delta V=V_2-V_1$ is produced. Finally, this corrected detection signal $\Delta V'=\gamma\times\Delta V$ is compared with the judgement standard upper and lower limits U.L. and L.L.

When the particle concentration is larger than the standard value, a larger number of particles are collected in the central portion 5 and therefore, the difference $\Delta V$ becomes larger. At the same time a large number of particles are deposited on the flat portion 3, so that the auxiliary signal $V_3$ from the light receiving element 21 becomes smaller than the standard value $V_{30}$. Therefore, the coefficient $\gamma$ becomes smaller than unity ($\gamma$<1) and thus the product $\Delta V'=\gamma\times\Delta V$ approaches the correct detection signal value. Similarly, when the particle concentration is too small, although the difference $\Delta V$ becomes smaller than the correct value, the product $\Delta V'$ approaches the correct difference value, because the signal $V_3$ becomes large and the coefficient $\gamma$ becomes larger than unity.

In this manner, by utilizing the auxiliary signal $V_3$ from the light receiving element 21 which receives the image of the flat bottom portion 3, it is possible to compensate for the variation of the particle concentration and/or total quantity of particles, and thus an accurate judgement can be carried out without being affected by the said variation.

Example 3

This example is a hybrid method between the above mentioned two examples. That is to say, the auxiliary signal $V_3$ is first compared with the upper and lower reference values $DR_1$ and $DR_2$, and when $V_3$<$DR_1$ or $V_3$>$DR_2$ the product $\Delta V'$ is used for judgement. But, when $DR_1\leqq V_3\leqq DR_2$, $\Delta V$ is used for determining the particle pattern.

The present invention is not limited to the embodiments explained above. In the above embodiment, the image of the bottom interior surface of the reaction vessel is formed on the light detector, but it is also possible to receive separately the light fluxes passing through the flat bottom surface portion, inclined bottom surface portion and lowermost central portion. Further, in the above embodiment the main bottom portion 4 is formed conically, but it may be shaped in some other form such as a pyramid, triangular prism or sphere. Moreover, the display device may be replaced by a printer.

**Claims**

1. A method of judging the occurrence of a particle agglutination reaction in a test liquid sample, wherein the sample is placed in a reaction vessel (1) having a main interior bottom surface portion that is inclined to the horizontal, and photoelectric means (15—18) are used to produce a detection signal which is dependent upon the difference between deposited particle concentration at a lowermost part (5) of the said bottom surface portion and deposited particle concentration at a higher part (4) of that surface portion and which is used as a basis for the said judgement, characterised in that the reaction vessel has a further interior bottom surface portion (3), which extends in a horizontal plane, photoelectric means are used to produce an auxiliary signal ($V_3$) which is dependent upon deposited particle concentration at the said further bottom surface portion, and the said auxiliary signal is used to control the making of the said judgement.

2. A method as claimed in claim 1, wherein an image of the particles on the said further bottom surface portion (3) is formed on a light receiving element (21) whereby the said auxiliary signal ($V_3$) is produced.

3. A method as claimed in claim 1, wherein a light flux transmitted through the said further bottom surface portion (3) is received by a light receiving element (21) whereby the said auxiliary signal ($V_3$) is produced.

4. A method as claimed in claim 1 or 2, wherein images of the particles deposited on the said higher part (4) and on the said lowermost part (5) of the said main bottom surface portion are projected respectively onto two separate light receiving elements (19, 20) which produce respective photoelectrically converted outputs ($V_1$, $V_2$), and the said detection signal is produced by deriving a difference between those two outputs.

5. A method as claimed in claim 1 or 3, wherein light fluxes transmitted through the said higher part (4) and the said lowermost part (5) of the said main bottom surface portion are received respectively by two separate light receiving elements (19, 20) which produce respective photo-electrically converted outputs ($V_1$, $V_2$), and the said detection signal is produced by deriving a difference between those two outputs.

6. A method as claimed in any one of claims 1 to 5, wherein when the said auxiliary signal ($V_3$) is within predetermined upper and lower values the occurrence of a particle agglutination reaction is judged by comparing the said detection signal with standard values.

7. A method as claimed in any one of claims 1 to 5, wherein a correction coefficient is calculated from the said auxiliary signal ($V_3$), the said detection signal is multiplied by the said correction coefficient to produce a corrected detection signal, and the occurrence of a particle agglutination reaction is judged by comparing the said corrected detection signal with standard values.

8. A method as claimed in claim 7, wherein said coefficient is derived by dividing the auxiliary signal ($V_3$) by a predetermined standard value thereof.

9. A reaction vessel, for use in judging the occurrence of a particle agglutination reaction in a test liquid sample contained in the vessel, having a main interior bottom surface portion that is inclined to the horizontal for receiving a pattern of deposited particles which is indicative of the degree of agglutination; characterised in that the vessel has a further interior bottom surface portion (3), which extends in a horizontal plane, for receiving deposited particles to afford an indication of particle concentration and/or the total quantity of particles in the test liquid sample.

10. A reaction vessel as claimed in claim 9, wherein the said main surface portion has a number of regularly-spaced small projections and/or depressions.

11. A reaction vessel as claimed in claim 9 or 10, wherein the said further bottom surface portion (3) adjoins the said main bottom surface portion at the uppermost edge thereof.

12. A reaction vessel as claimed in claim 9, 10 or 11, wherein the said main bottom surface portion is generally conical in form.

13. A reaction vessel as claimed in claim 9, 10, 11 or 12, comprising part of a plate (12) in which a recess is formed to provide the interior of the vessel, the said recess being one of a plurality of such recesses formed so that the plate provides a matrix of such vessels constructed integrally with one another.

**Patentansprüche**

1. Verfahren zur Beurteilung des Auftretens einer Agglutinationsreaktion in einer flüssigen Untersuchungsprobe, bei dem die Probe in ein Reaktionsgefäß (1) mit einem inneren, gegen die Horizontale geneigten Haupt-Bodenflächenabschnitt eingebracht wird und bei dem photoelektrische Mittel (15—18) zur Erzeugung eines Erfassungssignals verwendet werden, das von der Differenz zwischen der Konzentration der abgelagerten Teilchen an einem tiefsten Teil (5) des Haupt-Bodenflächenabschnitts und derjenigen an einem höheren Teil (4) des Haupt-Bodenflächenabschnitts abhängt und als Basis für die Beurteilung dient, dadurch gekennzeichnet, daß das Reaktionsgefäß einen zusätzlichen inneren Bodenflächenabschnitt (3) in einer Horizontalebene aufweist, photoelektrische Mittel zur Erzeugung eines Hilfssignals ($V_3$) verwendet werden, das von der Konzentration der auf dem zusätzlichen Bodenflächenabschnitt abgelagerten Teilchen abhängt, und das Hilfssignal zur Kontrolle der Durchführung der Beurteilung dient.

2. Verfahren nach Anspruch 1, bei dem ein Abbild der Teilchen auf dem zusätzlichen Bodenflächenabschnitt (3) auf einem Lichtempfangselement (21) gebildet wird, wodurch das Hilfssignal ($V_3$) erzeugt wird.

3. Verfahren nach Anspruch 1, bei dem ein durch den zusätzlichen Bodenflächenabschnitt (3) übertragener Lichtstrom von dem Lichtempfangselement empfangen wird, wodurch das Hilfssignal ($V_3$) erzeugt wird.

4. Verfahren nach Anspruch 1 oder 2, bei dem Abbilder der auf dem höheren Teil (4) abgelagerten Teilchen sowie der auf dem tiefsten Teil (5) des Haupt-Bodenflächenabschnitts abgelagerten Teilchen auf zwei separate jeweilige Lichtempfangselemente (19, 20) projiziert werden, die unterschiedliche photoelektrisch umgeformte Ausgangssignale ($V_1$, $V_2$) erzeugen, und das Erfassungssignal durch Ableiten einer Differenz zwischen diesen beiden Ausgangssignalen erzeugt wird.

5. Verfahren nach Anspruch 1 oder 3, bei dem durch den höheren Teil (4) und durch den tiefsten Teil (5) des Haupt-Bodenflächenabschnitts übertragene Lichtströme durch zwei separate jeweilige Lichtempfangselemente (19, 20) empfangen werden, die unterschiedliche photoelektrisch umgeformte Ausgangssignale ($V_1$, $V_2$) erzeugen, und das Erfassungssignal durch Ableiten einer Differenz zwischen diesen beiden Ausgangssignalen erzeugt wird.

6. Verfahren nach einem der Ansprüche 1 bis 5, bei dem im Fall des Auftretens des Hilfssignals ($V_3$) innerhalb vorbestimmter unterer und oberer Werte das Auftreten der Agglutinationsreaktion durch Vergleich des Erfassungssignals mit Standardwerten beurteilt wird.

7. Verfahren nach einem der Ansprüche 1 bis 5, bei dem ein Korrekturkoeffizient aus dem Hilfs-signal ($V_3$) berechnet wird, das Erfassungssignal mit dem Korrekturkoeffizienten zwecks Erzeugung eines korrigierten Erfassungssignals multipliziert wird und das Auftreten der Agglutinationsreaktion durch Vergleich des korrigierten Erfassungssignals mit Standardwerten beurteilt wird.

8. Verfahren nach Anspruch 7, bei dem der Korrekturkoeffizient durch Dividieren des Hilfs-signals ($V_3$) durch einen hierfür vorbestimmten Standardwert abgeleitet wird.

9. Reaktionsgefäß zur Verwendung bei der Beurteilung des Auftretens einer Agglutinationsreaktion in einer flüssigen Untersuchungsprobe in dem Reaktionsgefäß, mit einem gegenüber der Horizontalen geneigten Haupt-Bodenflächenabschnitt zum Aufnehmen eines Musters abgelagerter Teilchen als Maß für die Agglutination, dadurch gekennzeichnet, daß das Reaktionsgefäß einen zusätzlichen inneren Bodenflächenabschnitt (3), der sich in einer Horizontalebene erstreckt, aufweist, zum Aufnehmen abgelagerter Teilchen zum Erreichen einer Anzeige der Teilchenkonzentration und/oder der Gesamtmenge der Teilchen in der zu untersuchenden flüssigen Probe.

10. Reaktionsgefäß nach Anspruch 9, bei dem der Haupt-Bodenflächenabschnitt mehrere regelmäßig beabstandet angeordnete Vorsprünge und/oder Vertiefungen aufweist.

11. Reaktionsgefäß nach Anspruch 9 oder 10, bei dem der zusätzliche Bodenflächenabschnitt (3) an dem obersten Rand des Haupt-Bodenflächenabschnitts angrenzt.

12. Reaktionsgefäß nach Anspruch 9, 10 oder 11, bei dem der Haupt-Bodenflächenabschnitt allgemein konisch ist.

13. Reaktionsgefäß nach Anspruch 9, 10, 11 oder 12, das den Teil einer Platte (12) umfaßt, in die eine Vertiefung zur Bildung des Inneren des Reaktionsgefäßes ausgebildet ist, wobei die Vertiefung eine von mehreren solcher Vertiefungen ist, so daß die Platte eine Matrix von miteinander einstückig angeordneten Reaktionsgefäßen erreicht.

**Revendications**

1. Procédé d'évaluation de l'apparition d'une réaction d'agglutination de particules dans un échantillon liquide d'essai, dans lequel l'échantillon est placé dans un vase à réaction (1) ayant une partie de surface de fond intérieure principale qui est inclinée par rapport à l'horizontale et un dispositif photoélectrique (15—18) utilisé pour produire un signal de détection qui dépend de la différence entre la concentration des particules déposées à la partie inférieure (5) de ladite partie de surface de fond et la concentration de particules déposées dans une partie plus élevée (4) de cette partie de surface, et qui est utilisé comme une base pour ladite évaluation, procédé caractérisé en ce que le vase à réaction comporte une autre partie de surface de fond intérieure (3) qui s'étend dans un plan horizontal, un dispositif photoélectrique étant utilisé pour produire un signal auxiliaire ($V_3$) qui dépend de la concentration des particules déposées sur ladite autre partie de surface de fond, et ledit signal auxiliaire étant utilisé pour commander l'établissement de ladite évaluation.

2. Procédé selon la revendication 1, dans lequel une image des particules sur ladite autre partie de surface de fond (3) est formée sur un élément de réception de lumière (21) de manière que ledit signal auxiliaire ($V_3$) soit produit.

3. Procédé selon la revendication 1, dans lequel un flux lumineux transmis par ladite autre partie de surface de fond (3) est reçu par un élément de réception de lumière (21) de manière que ledit signal auxiliaire ($V_3$) soit produit.

4. Procédé selon la revendication 1 ou 2, dans lequel des images des particules déposées sur ladite partie plus élevée (4) et sur ladite partie inférieure (5) de ladite partie de surface de fond principale sont projetées respectivement sur deux éléments de réception de lumière séparés (19, 20) qui produisent des signaux de sortie respectifs convertis photoélectriquement ($V_1$, $V_2$) et ledit signal de détection étant produit en déterminant la différence entre ces deux signaux de sortie.

5. Procédé selon la revendication 1 ou 3, dans lequel des flux lumineux transmis par ladite partie plus élevée (4) et par ladite partie inférieure (5) de ladite partie de surface de fond principale sont reçus respectivement par deux éléments de réception de lumière séparés (19, 20) qui produisent des signaux de sortie respectifs ($V_1$, $V_2$) convertis photoélectriquement, et ledit signal de détection étant produit en déterminant la différence entre ces deux signaux de sortie.

6. Procédé selon l'une quelconque des revendications 1 à 5, dans lequel, si le signal auxiliaire ($V_3$) se situe dans des valeurs supérieure et inférieure prédéterminées, l'apparition d'une réaction d'agglutination de particules est évaluée en comparant ledit signal de détection avec des valeurs standard.

7. Procédé selon l'une quelconque des revendications 1 à 5, dans lequel un coefficient de correction est calculé à partir dudit signal auxiliare ($V_3$), ledit signal de détection est multiplié par ledit coefficient de correction pour produire un signal de détection corrigé et l'apparition d'une réaction d'agglutination de particules est évaluée en comparant ledit signal de détection corrigé avec des valeurs standard.

8. Procédé selon la revendication 7, dans lequel ledit coefficient est déterminé en divisant le signal auxiliaire ($V_3$) par sa valeur standard prédéterminée.

9. Vase à réaction destiné à évaluer l'apparition d'une réaction d'agglutination de particules dans un échantillon liquide d'essai que contient le vase, comprenant une partie de surface de fond intérieure principale qui est inclinée par rapport à l'horizontale pour recevoir une distribution de particules déposées qui indique le degré d'agglutination; caractérisé en ce que le vase comporte une autre partie de surface de fond intérieure (3) qui s'étend dans un plan horizontal pour recevoir des particules déposées afin de donner une indication sur une concentration de particules et/ou la quantité totale des particules dans l'échantillon liquide d'essai.

10. Vase à réaction selon la revendication 9, dans lequel ladite partie de surface principale comporte un certain nombre de petites parties en saillie et/ou en creux régulièrement espacées.

11. Vase à réaction selon la revendication 9 ou 10, dans lequel ladite autre partie de surface de fond intérieure (3) est contigüe à ladite partie de surface de fond principale à son bord supérieur.

12. Vase à réaction selon la revendication 9, 10 ou 11, dans lequel ladite partie de surface de fond principale a une forme générale conique.

13. Vase à réaction selon la revendication 9, 10, 11 ou 12, comprenant une partie d'une plaque 12 dans laquelle un logement est formé pour constituer l'intérieur du vase, ledit logement étant l'un de plusieurs de ces logements formés de manière que la plaque constitue une matrice de ces vases, réalisés solidairement les uns des autres.

## FIG_1

## FIG_2

# FIG. 3

| | | |
|---|---|---|
| $V_3$ | $V_2$ | $V_1$ |

A/D Converter 25

Computer 26

Display 28

Keyboard 27